# EUROPEAN PATENT SPECIFICATION

(11) **EP 2 144 861 B1**
(45) Date of publication and mention of the grant of the patent: **20.03.2013**
(21) Application number: 08745381.7
(22) Date of filing: 09.04.2008
(51) Int. Cl.: C07C 1/26, C07C 17/10

(54) **STABILIZATION OF CHLOROPROPENES**
STABILISIERUNG VON CHLORPROPENEN
STABILISATION DE CHLOROPROPÈNES

(30) Priority: 11.04.2007 US 911214 P
(43) Date of publication of application: 20.01.2010
(73) Proprietor: OCCIDENTAL CHEMICAL CORPORATION, Dallas, TX 75244 (US)
(72) Inventor: NYBERG, Janice, M., Wichita, Kansas 67212 (US)
(74) Representative: Albrecht, Thomas
(86) International application number: PCT/US2008/059762
(87) International publication number: WO 2008/127940

(56) References cited:
- EP-A- 1 116 706
- WO-A-02/100809
- US-A- 3 676 507
- US-A- 3 852 367
- DATABASE WPI Week 200170 Thomson Scientific, London, GB; AN 2001-609592 XP002488554 & JP 2001 206880 A (SUMITOMO CHEM CO LTD) 31 July 2001 (2001-07-31)
- DATABASE WPI Week 198236 Thomson Scientific, London, GB; AN 1982-75461E XP002488555 & JP 57 123126 A (ADEKA ARGUS IND KK) 31 July 1982 (1982-07-31)

## Description

### TECHNICAL FIELD

This invention relates to halogenated alkene moieties.

### BACKGROUND

Tetrachloropropenes are useful as precursors to fluorinated propenes used in manufacturing fluorinated blowing agents and refrigerants. Chlorinated organic compounds, including tetrachloropropenes, do however typically break down in prolonged contact with, singularly or in combination, heat, light, air, humidity or metals. Oxidation is a major mechanism of decomposition. US3959367 identifies the oxidation products of 1,1,2,3 tetrachloropropene as including primarily 1,1,1,3 tetrachloropropanone along with chloroacetyl chloride. Lab studies have shown the presence of tetrachloropropanone and increased acidity and phosgene levels upon storage of unstabilized 1,1,2,3 tetrachloropropene. EP0309958 describes stabilized C₃ unsaturated organic chlorine compounds including dichlorinated propenes.

JP 2001 206880 A relates to a method of preparing a halogenoallylfurfuryl alcohol which comprises reacting a furfural with a dihalogenated compound in water or a mixed solvent of water and an organic solvent in the presence of zinc and a phenol compound. In addition, it discloses a composition of 2,3-dichloropropene and para-tert-butylcatechol.

JP 57 123126 A relates to a stabilised allyl chloride composition containing an organic tin compound. In particular, it discloses a composition comprising allyl chloride and a phenol (cresol or hydroquinone).

US-A-3 676 507 discloses in its examples and claims 1 to 3 a composition of a stabilised trichloroethylene in the presence of para-methoxy phenol or p-tert-amyl phenol and synergistic combinations of two phenols.

US-A-3 852 367 discloses a composition of trichloroethylene or tetrachloroethylene stabilised against decomposition by heat, light, air, moisture and metals by the addition of phenols, like para-methoxyphenol.

WO 02/100809 A discloses in its claims 1, 6 and 9 a composition of 1,1,1,3-tetrachloropropane stabilised by para-methoxyphenol.

EP-A-1 116 706 discloses in its claim 1 a composition of 1,1,3,3-tetrachloropropene or 1,3,3,3-tetrachloropropene in the reaction with HF.

### SUMMARY

The inventors have discovered that tetrachloropropenes can be stabilized using an antioxidant, for example a phenolic antioxidant.

In one aspect, there is provided a composition comprising a chlorinated propene and a compound selected from the group of *p*-methoxyphenol and *p-tert-*amylphenol.

In some embodiments, the composition also includes an additional phenol compound selected from the group consisting of isopropyl-meta cresol (thymol), 4,4'-methylenebis(2,6-di-*tert*-butyl-phenol); 4,4'-bis(2,6-di-*tert*-butylphenol); 2,2-biphenyldiols, 4,4-biphenyldiols; derivatives of 2,2- and 4,4-biphenyldiols; 2,2'-methylenebis(4-ethyl-6-*tert*-butylphenol); 2,2'-methylenebis(4-methyl-6-*tert*butylphenol); 4,4,-butylidenebis(3-metbyl-6-*tert*-butylphenol); 4,4-isopropylidenebis(2,6-di-*tert*-butylphenol); 2,2'-methylenebis(4-methyl-6-nonylphenol); 2,2'-isobutylidenebis(4,6-dimethylphenol); 2,2'-methylenebis(4-methyl-6-cyclohexylphenol); 2,6-di-*tert*-butyl-4-methyl-phenol; 2,6-di-*tert*-butyl-4-ethylphenol; 2,4-dimethyl-6-*tert*-butylphenol; 2,6-di-*tert*-α-dimethylamino-*p*-cresol; 2,6-di-*tert*-butyl-4-(*N,N'*-dimethylaminomethyl)phenol; 4,4'-thiobis(2-methyl-6-*tert*butylphenol); 4,4'-thiobis (3-methyl-6-*tert*-butylphenol); 2,2'-thiobis(4-methyl-6-*tert*butylphenol; tocopherol; hydroquinone; *tert*-butyl hydroquinone.

In some embodiments, the composition includes *p*-methoxyphenol, for example, from about 1 ppm to about 1000 ppm of *p*-methoxyphenol. In some embodiments, the composition is substantially free of *p*-*tert*-amylphenol.

In some embodiments, the composition includes *p*-*tert*-amylphenol, for example, from about 1 ppm to about 1000 ppm *p*-*tert*-amylphenol. In some embodiments, the composition is substantially free of *p*-methoxyphenol.

In some embodiments, the chlorinated propene is a tetrachloropropene, for example 1,1,2,3-tetrachloropropene or 2,3,3,3-tetrachloropropene or 1,1,3,3-tetrachloropropene or 1,3,3,3-tetrachloropropene or (*cis* or *trans*)-1,2,3,3-tetrachloropropene .

In some embodiments, the chlorinated propene is a trichloropropene, for example, 1,1,3-trichloropropene.

In some embodiments, the composition is substantially free of propanone and phosgene is <20 ppm.

In some embodiments, the temperature of the composition is from about 10 to about 200°C.

In some embodiments, the composition consists essentially of the chlorinated propene and the one or more phenolic compounds.

In some embodiments, the desired use is to react the stabilized chlorinated propene, for example, 1,1,2,3-tetrachloropropene to produce a fluorinated propene, for example 2,3,3,3-tetrafluoropropene, for example by reacting the composition with HF, such as in the presence of Cl₂ and SbCl₅. Accordingly in some of the embodiments, the composition further comprises HF. In some sub-embodiments thereof, the composition further comprises Cl₂ and SbCl₅. Embodiments thereof include those wherein the chlorinated propene is 1,1,2,3-tetrachloropropene.

In another aspect, there is provided an article comprising a container, for example a metal container, and a composition as described above contained within said container.

In some embodiments, the metal container is made of a metal, for example stainless steel or a MONEL^{®} metal. In some embodiments, the MONEL^{®} metal is in contact with the composition comprising the chlorinated propene.

In some embodiments, the container is a reaction vessel. In some embodiments, the container is a sealed container, for example, a sealed container is substantially free of oxygen and/or substantially free of water.

In some embodiments, the composition comprises *p*-methoxyphenol, for example, present in the composition in an amount from about 1 ppm to about 1000 ppm. In some embodiments, the composition comprises *p*-*tert*-amylphenol, for example, wherein the *p*-*tert*-amylphenol is present in the composition in an amount from about 1 ppm to about 1000 ppm.

In some embodiments the container is lined with a polymeric coating, for example a coating comprising a phenolic or epoxy resin. Embodiments thereof include those wherein the chlorinated propene is 1,1,2,3-tetrachloropropene and the lining serves to protect the chlorinated propene from possible exposure to a metal, for example carbon steel, that could lead to premature chemical breakdown of the chlorinated propene.

In one aspect, there is provided a method of making a fluorinated propene, the method comprising:
(a) providing a composition comprising chlorinated propene and a compound selected from the group consisting of *p*-methoxyphenol and *p-tert-*amylphenol or an embodiment thereof as described herein; and
(b) subjecting the composition to reaction conditions sufficient to effect a reaction wherein at least one chlorine atom of the chlorinated propene is substituted by fluorine to provide a fluorinated propene.

Embodiments of the method are those comprising reacting the chlorinated propene with HF. Embodiments of the method also include those comprising reacting the chlorinated propene with HF in the presence of chlorine and SbCl₅.

In some embodiments, the chlorinated propene is a tetrachloropropene, for example, 1,1,2,3-tetrachloropropene or 2,3,3,3-tetrachloropropene.

In some embodiments, the fluorinated propene formed is a tetrafluoropropene, for example, 2,3,3,3 tetrafluoropropene.

In some embodiments, the composition is in contact with a metal vessel. In some embodiments, the metal vessel comprises a MONEL^{®} metal.

In some embodiments, the antioxidants described herein can provide improved stability of tetrachloropropenes under a variety of conditions, for example storage conditions and/or processing conditions.

The details of one or more embodiments of the invention are set forth in the accompanying drawings and the description below. Other features, objects, and advantages of the invention will be apparent from the description and from the claims.

### BRIEF DESCRIPTION OF THE DRAWINGS

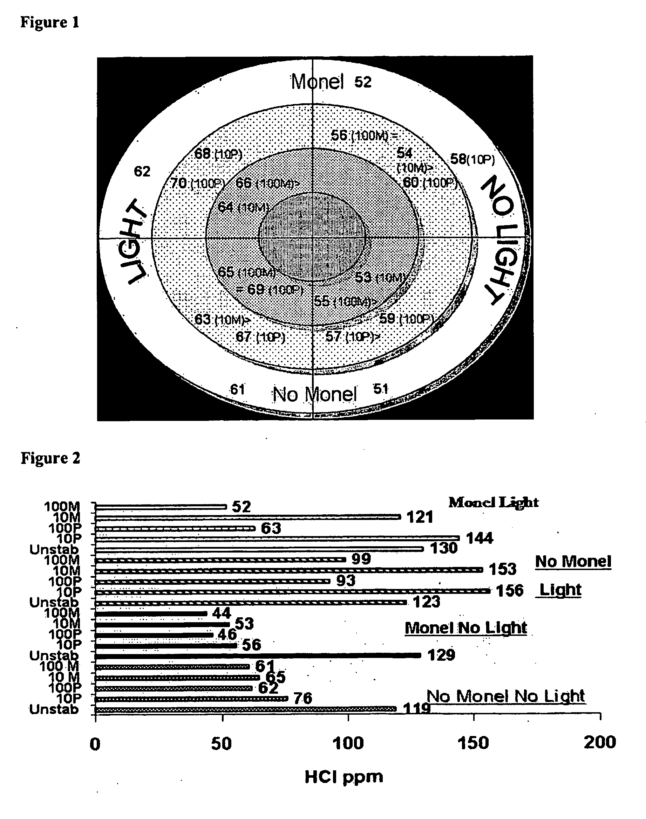

**Figure 1** shows color development in samples of 1,1,2,3 tetrachloropropene samples (identified by sample numbers) stored in the presence or absence of MONEL metal; and in the presence or absence of light (indicated by the quadrant of the diagram in which the sample number appears). The samples were either unstabilized (no parenthesis after the sample number) or stabilized with 10 ppm or 1000 ppm of MEHQ as indicated by parentheses following the sample number (10M and 100M mean 10 ppm and 100 ppm MEHQ stabilized material respectively while 10P and 100P mean 10 ppm and 100 ppm PTAP stabilized material respectively). Samples whose numbers are placed towards the center of the diagram showed more color development.
**Figure 2** shows acidity development (HCl concentration) in samples of 1,1,2,3 tetrachloropropene stored in the presence or absence of MONEL metal; and in the presence or absence of light. The top five bars are +Metal + Light; the next five are - Metal +Light; the next five are +Metal -Light; the bottom five are -Metal -Light. The samples were either unstabilized ("Unstab.") or stabilized with 10 ppm or 1000 ppm of MEHQ or PTAP (10M and 100M mean 10 ppm and 100 ppm MEHQ stabilized material respectively while 10P and 100P mean 10 ppm and 100 ppm PTAP stabilized material respectively). The value shown to the right of each bar is the HCl concentration in ppm.

### DETAILED DESCRIPTION

### I. Chlorinated Propenes

A "chlorinated propene" is a compound according to the following formula:

C(H)₃₋ₓ(Cl)ₓ-C(H)_{1-y}(Cl)_{y}=C(H)_{2-z}(Cl)_{z}

wherein:
x is 0, 1, 2, or 3;
y is 0 or 1; and
z is 0, 1, or 2;
provided that at least one of x, y, and z is other than zero.

Chlorinated propenes wherein z is one may exist in either of two geometrical isomeric forms (E and Z) (or as a mixture of such isomers).

Non-limiting examples of chlorinated propenes include those wherein at least one of y and z is other than zero, for example wherein y is I and/or z is 1 or 2. Other examples thereof include those wherein x is 0, 1, or 2.

Chlorinated propenes include: *E*-1-chloropropene, *Z*-1-chloropropene, 2-chloropropene, 3-chloropropene, 1,1-dichloropropene, *E*-1,2-dichloropropene, *Z*-1,2-dichloropropene, *E*-1,3-dichloropropene, *Z*-1,3-dichloropropene, 2,3-dichloropropcne, 3,3-dichloropropene, 1,1,2-trichloropropene, 1,1,3-trichloropropene, E-1,2,3-trichloropropene, *Z*-1,2,3-trichloropropene, *E*-1,3,3-trichloropropene, Z-1,3,3-trichloropropene, 2,3,3-trichloropropene, 3,3,3-trichloropropene, 1,1,2,3-tetrachloropropene, 1,1,3,3-tetrachloropropene, *E*-1,2,3,3-tetrachloropropene, Z-1,2,3,3-tetrachloropropene, *E*-1,3,3,3-tetrachloropropene, *Z*-1,3,3,3-tetrachloropropene, 2,3,3,3-tetrachloropropene, 1,1,2,3,3-pentachloropropene, 1,1,3,3,3-pentachloropropene, *E*-1,2,3,3,3-pentachloropropene, *Z*-1,2,3,3,3-pentachloropropene, and hexachloropropene.

### II. Compositions Comprising Chlorinated Propenes and a Phenolic Antioxidant

Oxidative degradation of components of a composition may be inhibited by including in the composition one or more compounds one or more compounds which have the property of inhibiting oxidation, or so-called "antioxidants". The selection of a suitable antioxidant for a given process from the broad range which are commercially available or otherwise known in the art can be very challenging due to a variety of factors involved such as, for example, different chemical processing conditions. In general, since an anti-oxidant will be an impurity in the composition in which it is included, it is desirable to select an antioxidant which is effective at low concentrations. In addition, antioxidants themselves may be oxidized over time to darkly colored oxidation products, which can lead to undesirable product appearance, or undesirable inconsistencies and changes in product appearance. Thus, it is advantageous if a product composition can be found wherein the problem of such color formation is minimized, which may be because, for example, the antioxidant is effective at very low concentrations, minimal degradation of the antioxidant itself occurs, or such degradation products as are formed with the particular antioxidant in the particular composition are not highly colored (or a combination of such factors).

The inventors have discovered that chlorinated propene containing compositions can be stabilized by including an effective amount of *p*-methoxyphenol ("MEHQ") or *p*-*tert*-amylphenol ("PTAP") in the composition as phenolic antioxidants. In some preferred embodiments, PTAP is a preferred stabilizer for a composition comprising a chlorinated propene. Any chlorinated propene as described above; or any combination of such chlorinated propenes, may be used in the compositions described herein.

In some embodiments one or more additional phenolic compounds may be included in the composition, for example, one or more phenols selected from the group consisting of isopropyl-meta cresol (thymol), 4,4'-methylenebis(2,6-di-*tert*butylphenol); 4,4'-bis(2,6-di-*tert*-butylphenol); 2,2-biphenyldiols, 4,4-biphenyldiols; derivatives of 2,2- and 4,4-biphenyldiols; 2,2'-methylenebis(4-ethyl-6-*tert*butylphenol); 2,2'-methylenebis(4-methyl-6-*tert*-butylphenol); 4,4-butylidcncbis(3-methyl-6-*tert*-butylphenol); 4,4,-isopropylidenebis(2,6-di-*tert*-butylphenol); 2,2'-methylenebis(4-methyl-6-nonylphenol); 2,2'-isobutylidenebis(4,6-dimethylphenol); 2,2'-methylenebis(4-methyl-6-cyclohexylphenol); 2,6-di-*tert*-butyl-4-methyl-phenol (BHT); 2,6-di-*tert*-butyl-4-ethylphenol; 2,4-dimethyl-6-*tert*-butylphenol; 2,6-di-*tert*-α-dimethylamino-*p*-cresol; 2,6-di-*tert*-butyl-4(N,N'-dimethylaminomethyl)phenol; 4,4'-thiobis(2-methyl-6-*tert*-butylphenol); 4,4'-thiobis (3-methyl-6-*tert*-butylphenol); 2,2'-thiobis(4-methyl-6-*tert*-butylphenol; tocopherol; hydroquinone; *tert*-butyl hydroquinone.

Chlorinated propenes include compounds having one, two, three, four, five, or six chlorine atoms, referred to as mono-, di-, tri-, tetra-, penta-, or hexa-chloropropenes respectively. Examples of chlorinated propenes include monochloropropenes, such as *E*-1-chloropropene, *Z*-1-chloropropene, 2-chloropropene, and 3-chloropropene, dichloropropenes, such as 1,1-dichloropropene, *E*-1,2-dichloropropene, Z-1,2-dichloropropene, *E*-1,3-dichloropropene, *Z*-1,3-dichloropropene, 2,3-dichloropropene, and 3,3-dichloropropene, trichloropropenes, such as 1,1,2-trichloropropene, 1,1,3-trichloropropene, *E*-1,2,3-trichloropropene, *Z*-1,2,3-trichloropropene, *E*-1,3,3-trichloropropene, *Z*-1,3,3-trichloropropene, 2,3,3-trichloropropene, and 3,3,3-trichloropropene, tetrachloropropenes, such as 1,1,2,3-tetrachloropropene, 1,1,3,3-tetrachloropropene, *E*-1,2,3,3-tetrachloropropene, *Z*-1,2,3,3-tetrachloropropene, *E-*1,3,3,3-tetrachloropropene, *Z*-1,3,3,3-tetrachloropropene, and 2,3,3,3-tetrachloropropene, pentachloropropenes, such as 1,1,2,3,3-pentachloropropene, 1,1,3,3,3-pentachloropropene, *E*-1,2,3,3,3-pentachloropropene, and *Z*-1,2,3,3,3-pentachloropropene, and hexachloropropene, any of which, or mixtures of any of which, may be used in the compositions described herein. Preferred tetrachloropropenes for use in the compositions of the invention include 1,1,2,3-tetrachloropropene and 2,3,3,3-tetrachloropropene.

The phenolic antioxidants described herein can be used to stabilize the chlorinated propenes during storage of a chlorinated propene-containing composition, or when the chlorinated propene-containing composition is being used in a process. Examples of processes where stabilization may be useful include manufacturing processes (e.g., a process preparing the chlorinated propene or a chemical transformation converting the chlorinated propene to a different chemical entity).

The amount of phenolic antioxidant which is present in the composition can be a storage stabilizing amount, i.e., an amount sufficient to substantially inhibit the decomposition of the chlorinated propene. The time for storage may be a short period of a few weeks or a longer period of up to one or two years. For example, the phenolic antioxidant such as MEHQ or PTAP can be present in an amount of at least I part per million parts of the composition (ppm), at least 2 ppm, at least 3 ppm, at least 4 ppm, at least 5 ppm, at least 6 ppm, at least 7 ppm, at least 8 ppm, at least 9 ppm, at least 10 ppm, at least 11 ppm, at least 12 ppm, at least 13 ppm, at least 14 ppm, at least 15 ppm, at least 20 ppm, at least 25 ppm, at least 30 ppm, at least 40 ppm, at least 50 ppm, at least 60 ppm, at least 70 ppm, at least 80 ppm, at least 90 ppm, at least 100 ppm. Thus suitable amounts of MEHQ or PTAP for inclusion in such compositions include, for example, about 1 ppm, about 2 ppm, about 3 ppm, about 4 ppm, about 5 ppm, about 10 ppm, about 15 ppm, about 20 ppm, about 25 ppm, about 30 ppm, about 40 ppm, about 50 ppm, about 100 ppm, or wherein the MEHQ or PTAP is present in an amount which is in a range between any two of these amounts. The preferred concentration of MEHQ or PTAP is in the range from about 5 ppm to about 100 ppm, for example from about 5 ppm to about 50 ppm, or from about 10 ppm to about 20 ppm. The ppm values represent the amount by weight relative to the amount by weight of the stabilized chlorinated propenes. In cases where more than one phenolic antioxidant is present, it may suffice for the total amount of the phenolic antioxidants present to be present in the amounts indicated.

In some embodiments, the phenolic antioxidant present in a composition described herein can be present in an amount sufficient to stabilize the chlorinated propene during a product processing or purification process. For example, the amount of phenolic antioxidant such as such as MEHQ or PTAP can be present in an amount less than about 1000 ppm.

It is generally preferred that the chlorinated propene containing compositions described herein are substantially free of breakdown products, i.e., those products indicative of a destabilized chlorinated propene. For example, it is generally preferred that the amount of breakdown products be less than about 5000 ppm e.g. less than about 4000 ppm, less than about 2000 ppm, less than about 1000 ppm, less than about 500 ppm, less than about 200 ppm, less than about 100 ppm, less than about 50 ppm, less than about 10 ppm. Exemplary breakdown products include propanones (e.g., tetrachloropropanone), HCl, and phosgene.

In some embodiments the stability of a composition can be evaluated by observing the composition. For example, formation of color in the composition generally indicates a chemical reaction such as a breakdown of the chlorinated propene or oxidation of the antioxidant has occurred. Similarly the presence of a coating on the metal or lined surface in contact with the chlorinated propene or deposited particles within the composition may also indicate reactivity of the composition, for example with air or a material to which the composition is exposed (e.g., a metal). Preferred compositions are those which display no indication of breakdown or reactivity of the composition during storage conditions. Preferred compositions according to the invention include those wherein the MEHQ or PTAP is used to stabilize essentially pure chlorinated propenes during storage. Accordingly, examples of such a composition are those wherein composition consists essentially of the chlorinated propene and the one or more phenolic compounds used as stabilizing agents. For example, suitable compositions include compositions consisting essentially of one or more chlorinated propenes and an amount of MEHQ and/or PTAP which is effective to stabilize the chlorinated propene during the desired storage period, for example an amount in the range from about 5 ppm to about 100 ppm, for example from about 5 ppm to about 50 ppm, for example about 5 ppm to about 20 ppm, for example about 10 ppm. An example of such a composition is one consisting essentially of 1,1,2,3-tetrachloropropene and an amount from about 5 ppm to about 100 ppm, for example from about 5 ppm to about 50 ppm, for example about 5 ppm to about 20 ppm, for example about 10 ppm of PTAP or MEHQ.

### III. Processing and Storage of Chlorinated Propene Containing Compositions The compositions described herein are preferably stable.

A composition described herein is generally stored in a container, such as a metal container or a metal container lined with a phenolic resin or a polypropylene container. The metal used for the container may be any metal having suitable physical characteristics (e.g. strength characteristics) and chemical characteristics (e.g. corrosion resistance). The metal selected should be one which will not react or be corroded by the composition stored in the container, particularly if the container is not lined and the composition is in contact with the walls of the container. Examples of suitable metals include stainless steels and alloys such as MONEL^{®} metals.

Accordingly, the compositions described herein, can be in long term contact with a metal such as a MONEL^{®} metal. MONEL^{®} is a trademark for a series of stainless metal alloys, primarily composed of nickel (up to 67%) and copper, with some iron and other trace elements. The alloys are resistant to corrosion and acids, and some of the alloys can withstand a fire in pure oxygen. The alloys are commonly used in applications with highly corrosive conditions. Small additions of aluminum and titanium form an alloy with the same corrosion resistance but with much greater strength. Illustrative compositions of suitable alloys are shown in Table 1.

**Table 1. MONEL^{®} Alloy Compositions**

| Alloy Trademark | Limiting Composition (%) | | | | | | | | | |
|---|---|---|---|---|---|---|---|---|---|---|
| | Ni | Co | C | Mn | Fe | S | Si | Al | Ti | Cu |
| MONEL Alloy 400 | ≥63 (Ni plus Co) | | ≤0.3 | ≤2.0 | ≤2.5 | ≤0.024 | ≤0.5 | - | - | 28-34 |
| MONEL Alloy 401 | 40-45 | ≤0.25 | ≤0.3 | ≤2.25 | ≤0.75 | ≤0.015 | ≤0.25 | - | - | Balance* |
| MONEL Alloy 404 | 52-57 (Ni plus Co) | | ≤0.15 | ≤0.10 | ≤0.50 | ≤0.024 | ≤0.10 | ≤0.05 | - | Balance* |
| MONEL Alloy R-405 | ≥63 (Ni plus Co) | | ≤0.3 | ≤2.0 | ≤2.5 | 0.025-0.060 | ≤0.5 | - | - | 28-34 |
| MONEL Alloy K-500 | ≥63 (Ni plus Co) | | ≤0.25 | ≤1.5 | ≤2.0 | ≤0.01 | ≤0.5 | 2.30-3.15 | 0.35-0.85 | 27-33 |

| | | | | | | | | | | |
|---|---|---|---|---|---|---|---|---|---|---|
| *Balance means the element predominates the balance. Source: Special Metals Corporation, Publications Nos. SMC-053 (Feb. 2005), SMC-084 (Sep. 2004), SMC-059 (Sep. 2004), SMC-085 (Sep. 2004), SMC-062 (Sep. 2004). | | | | | | | | | | |

The compositions described herein can be in long term contact with a tank liner with a phenolic or epoxy resin liner such as Plasite 3070 (bake coating using an unmodified phenolic resin with superior resistance to acids and solvent available from Carboline Company described in the Product Data Sheet published August 2003 by Carboline Company), Plasite 7122 (cross-linked epoxy phenolic coating cured with an alkaline curing agent formulated with a wide range of chemical resistance available from Carbolinc Company described in the Product Data Sheet published May 2007 by Carboline Company), Plasite 9052 (water-resistant epoxy coating polymerized with a polyamine-type curing agent available from Carboline Company described in the Product Data Sheet published August 2003 by Carboline Company) or a non-baked liner such as Plasite 9122L (a cross linked epoxy-phenolic cured with a polyamine curing agent available from Carboline Company described in the Product Data Sheet published August 2003 by Carboline Company). In some embodiments, a composition described herein is stored in a sealed container. For example, a container including a composition described herein can be sealed, i.e. closed to the atmosphere. In some embodiments, the container is purged with an inert gas prior to sealing. In some instances, the container is removed of all or substantially all water and/or water vapor prior to sealing.

A composition can be stored for a variety of lengths of time, such as 1 day, 2 days, 3 days, 4 days, 5 days, 1 week, 2 weeks, 1 month, 2 months, 6 months, 1 year or longer. The storage conditions can vary. For example, the sample can be exposed to light or can be stored in a light blocking container. Storage temperatures will typically be at ambient conditions and generally range from about 0 °C to about 45 °C. In some embodiments, the process of adding the stabilizer can be accomplished in a variety of methods. In one application, the stabilizer can be dissolved directly into the process vessel. A possible improvement would be to dissolve the stabilizer in a dedicated vessel and inject controlled quantities into a process vessel for stabilization. In yet another application, MEHQ or PTAP can be dissolved to a concentration of less than 0.045 Wt.%, less than 0.040 Wt.%, less than 0.035 Wt.%, less than 0.030 Wt.%, less than 0.025 Wt.%, less than 0.020 Wt.%, less than 0.015 Wt.%, less than 0.010 Wt.% in 1,1,2,3 tetrachloropropene. The mixture can be injected into the process at the point of producing purified 1,1,2,3-tetrachloropropene to provide chemical stability.

In some embodiments, the compositions described herein require stability during a reaction process, for example, conversion of a chlorinated propene to a fluorinated propene. However, in some embodiments, the conversion process may require very low stabilizer concentration in order to prevent fouling or inactivation of the process catalyst. In some preferred embodiments, a composition including 1,1,2,3-tetrachloropropene or 2,3,3,3-tetrachloropropene can include 10 ppm or lesser amounts of PTAP or MEHQ in instances where the 1,1,2,3-tetrachloropropene or 2,3,3,3-tetrachloropropene will be used in a reaction to be converted to 2,3,3,3-tetrafluoropropene.

In some embodiments, the stabilizer concentration of the tetrachloropropene precursor to the tetrafluoropropene may need to be at higher concentrations ranging from greater than 10 ppm and as high as 1000 ppm in order to minimize phosgene formation In those instances, the stabilizer may need to be removed through contact with a caustic solid bed or various molecular sieves prior to feeding the tetrachloropropene to the production process. In some embodiments, the stabilized 1,1,2,3-tetrachloropropene could be fed into a hydrofluorination reactor with the desired intention of producing 2,3,3,3-tetrafluoropropane. A better option is to feed hydrofluoric acid (HF) and chlorine into a liquid phase hydrofluorination reactor with an antimony pentachloride (SbCl₅) catalyst to produce the desired 2,3,3,3-tetrafluoropropene. Operating conditions could be less than 1.03 x 10⁶ Pa (150 psig), or less than 1.89 x 10⁵ Pa (100 psig), or less than 5.2 x 10⁵ Pa (75 psig). Operating temperatures could be less than 149°C (300°F), or less than 121°C (250°F), or less than 98.9°C (210°F), or less than 93.3°C (200°F), or less than 87.8°C (190°F), or less than 82.2°C (180°F).

### IV. Examples

### Example 1. Effect of Stabilizer Choice and Temperature on HCl and Phosgene Formation in 1,1,2,3 Tetrachloropropene

Duplicate samples containing 10 ppm **MEHQ** or 10 ppm PTAP or unstabilized product were stored in the dark at 25 °C or 55 °C for a period of 129 days. No metal coupons were present. Starting material from lab distillations, had between 200 ppm to 300 ppm acidity as HCl and 99.7% purity. No efforts were made during product sampling to exclude exposure to air. After the sample aliquots were placed into the storage sample bottles the headspace was purged with nitrogen prior to closure. Table I shows the analytical results.

**Table 2. Effect of Stabilizer Choice and Temperature on HCl and Phosgene Formation in 1,1,2,3 Tetrachloropropene with No Metal, No Light**

| **(a) Storage at 25 °C** | | | | | | |
|---|---|---|---|---|---|---|
| | **Unstabilized** | **10.5 ppm MEHQ** | **10 ppm PTAP** | **Unstabilized** | **10.5 ppm MEHQ** | **10 ppm PTAP** |
| **Storage Days** | **COCl₂, ppm** | **COCl₂, ppm** | **COCl₂, ppm** | **HCl, ppm** | **HCl, ppm** | **HCl, ppm** |
| 0 | 30 | 0 | 16 | 263 | 221 | 203 |
| 7 | 62 | 13 | 17 | 279 | 236 | 236 |
| 14 | 49 | 14 | 12 | 303 | 224 | 233 |
| 33 | 84 | 13 | 18 | 300 | 226 | 225 |
| 59 | 100 | 16 | 21 | 309 | 222 | 217 |
| 90 | 100 | 26 | 34 | 340 | 252 | 251 |
| 129 | 107 | 29 | 39 | 336 | 251 | 257 |

| **(b) Storage at 55 °C** | | | | | | |
|---|---|---|---|---|---|---|
| | **Unstabilized** | **10.5 ppm MEHQ** | **10 ppm PTAP** | **Unstabilized** | **10.5 ppm MEHQ** | **10 ppm PTAP** |
| **Storage Days** | **COCl₂, ppm** | **COCl₂, ppm** | **COCl₂, ppm** | **HCl, ppm** | **HCl, ppm** | **HCl, ppm** |
| 0 | 30 | 15 | 16 | 263 | 221 | 203 |
| 7 | 85 | 15 | 21 | 346 | 254 | 265 |
| 14 | 69 | 14 | 22 | 317 | 240 | 251 |
| 33 | 90 | 16 | 22 | 380 | 251 | 275 |
| 59 | 151 | 16 | 32 | 417 | 272 | 312 |
| 90 | 144 | 26 | 54 | 528 | 333 | 367 |
| 129 | 169 | 31 | 83 | 525 | 313 | 435 |

The highest HCl values were seen in the unstabilized samples stored at the higher 55 °C temperature. The highest HCl value seen was 525 ppm as HCl. At ambient temperature, little difference in HCl values was seen for stabilized or unstabilized samples. Comparisons of phosgene values shown are not necessarily reliable due to delays in obtaining GC aliquots of the samples and actual analysis. Later studies showed that air contamination of the some of the samples may have caused some phosgene values to be artificially higher.

Color development varied among the samples. All unstabilized samples showed no color formation over the storage time, even at elevated temperature. PTAP stabilized samples stored at ambient temperature remained colorless over the storage time whereas MEHQ stabilized material started developing some color after 14 days of storage. At elevated temperature, both MEHQ and PTAP developed some color at 14 days storage but again PTAP showed less color than MEHQ.

### Example 2 Effect of MONEL^{®} Metal, Stabilizer Choice and Concentration and Light on 1,1,2,3 Tetrachloropropene Product Color at Ambient Temperature

The study was conducted for a total of 166 days using 1,1,2,3 tetrachloropropene with 99.9% purity. The container had been purged with nitrogen just before sample collection and the headspace was swept with nitrogen before closing the container. The MONEL^{®} alloy 400 coupons used were scrubbed with a plastic scrubber and soap and water and then rinsed with de-ionized water. They were heated in a 100 °C oven for one hour and cooled in a desiccator prior to use. Duplicate samples of 0, 10 or 100 ppm PTAP or MEHQ stabilizer in 1,1,2,3 tetrachloropropene were stored at 25 °C with or without the presence of MONEL^{®} metal coupons and with or without light. Starting acidity of stock solution was 48 ppm HCl. The samples were nitrogen padded prior to storage.

Visual color observations were made periodically over the storage period. Figure 1 shows the relative color changes observed among the samples following storage. The two-digit number represents the sample number. The value in parenthesis behind the number represents the stabilizer concentration in ppm. M means MEHQ and P means PTAP. If there is parenthesis behind the number, the sample is unstabilized. The outer circle means that samples 61, 62, 52 and 58 (10P) remained colorless throughout the study. The different fill patterns shown in the concentric circle regions indicate that the samples located closer to the center were observed to have become darker in color relative to those further from the center. Where less than or equal signs are shown they represent the color relative to other samples in that color range. For example, 56 (100M) = 54 (10M) > 60 (100P) shown in the top right quadrant, means that sample numbers 56 and 54 appeared to be the same color while 60 was lighter in color compared to 56 and 54. Location in the top right quadrant also indicates that these samples were stored with no light present and MONEL^{®} metal present.

All unstabilized samples showed no color development. Of the stabilized samples, conditions of light and MONEL^{®} metal present resulted in the least color development at 98 days storage. However, at 140 and 166 days storage, more samples with MONEL^{®} metal present and no light present showed the least color development. All MEHQ samples developed color whereas overall PTAP stabilized material had one sample with no color development and less color than the MEHQ samples.

At the end of the 166 days storage, HCl values were measured to determine overall stability under the storage conditions. Figure 2 shows a graph of the acidity values for the various types of samples with the measured HCl values (in ppm) indicated by the numeral to the right of each bar. Again, 10M and 100M mean 10 ppm and 100 ppm MEHQ stabilized material respectively, 10P and 100P mean 10 ppm and 100 ppm PTAP stabilized material respectively, while "Unstab." indicates unstabilized material (no MEHQ or PTAP). Different bar fill patterns correspond to particular storage conditions of samples (with/without MONEL^{®} metal; with/without light).

The maximum acidity value seen for unstabilized samples was 130 ppm HCl. When light was present, the 100 ppm stabilizer concentrations for MEHQ and PTAP were required to suppress HCl formation as compared to unstabilized material. Under these conditions, PTAP at 100 ppm concentration would be the better choice to minimize color formation in the stored product. In the absence of light, 10 ppm MEHQ or 10 ppm PTAP did achieve lower HCl values than unstabilized material.

### Example 3. Effect of MEHQ and PTAP Stabilizing 1,1,3,3-Tetrachloropropene Stored Under Various Conditions

1,1,3,3-Tetrachloropropene compositions were subjected to a variety of storage conditions and evaluated for evidence of breakdown or reactivity of the composition. MEHQ and PTAP were used to stabilize the 1,1,3,3-tetrachloropropene containing compositions.

The 1,1,3,3 tetrachloropropene samples were stored in a lighted oven at 55 °C over a 90-day period. Stabilizer levels included unstabilized, 75 ppm MEHQ, 150 ppm MEHQ and 300 ppm MEHQ. Some samples also included MONEL^{®} alloy 400 coupons, some using a nitrogen pad and some with air present. At various time points, the acidity (HCl content) of the samples was measured and the appearance of the MONEL^{®} alloy coupons observed. The results of the experiments are shown in Table 3.

**Table 3 - Storage Stability Studies with MEHQ in 1,1,3,3- Tetrachloropropene**

| Stabilizer | % HCl | | | | Coupon Appearance | |
|---|---|---|---|---|---|---|
| | Day 0 | Day 30 | Day 69 | Day 90 | Day 30 | Day 69 |
| Unstabilized | 0.0124 | 0.0223 | 0.0278 | 0.0347 | | |
| 75 ppm MEHQ | 0.0149 | 0.0047 | 0.0092 | 0.0089 | | |
| 150 ppm MEHQ | 0.0131 | 0.0092 | 0.0099 | 0.0092 | | |
| 300 ppm MEHQ | 0.0139 | 0.0119 | 0.0104 | 0.0104 | | |
| Unstabilized with Monel, N₂ Pad | 0.0124 | 0.1253 | N/A | N/A | Greenish coating, easily removed | N/A |
| Unstabilized Monel Air | 0.0124 | 0.0248 | 0.0419 | N/A | Whitish coating, easily removed | Greenish deposit, easily removed with water. Solution yellow linge. |
| 150 ppm MEHQ Monel Air | 0.0131 | 0.0097 | 0.0102 | N/A | Green coating, required HCl to remove. Solution slightly pink | Greenish deposit easily removed with water. Solution yellow linge |
| 300 ppm MEHQ Monel Air | 0.0139 | 0.0119 | 0.0122 | N/A | Greenish brown coating, required HCl to remove. Solution vibrant pink. | Definite Monel attack, HCl required to remove, Solution Vibrant Pink |

As little as 75 ppm MEHQ was sufficient to limit HCl levels for samples with no metal added over 90 days storage while the unstabilized material increased in HCl from 0.0124 to 0.0347% over the same period. Similar results were seen in the presence of MONEL^{®} alloy with or without a nitrogen pad. Deposits were observed on the MONEL^{®} alloy coupons over storage time. HCl was required to remove the deposits in samples containing MEHQ. In fact, the higher levels of MEHQ contributed to heavier and more difficult to remove deposits and a pinkish color in the solution. At longer storage time, visual metal corrosion was observed.

A similar storage stability study was conducted using lower concentrations of MEHQ at 10, 20 and 50 ppm and no metals present, the results of which are provided in Table 4 below. Samples with 20 ppm PTAP, 300 ppm PTAP and unstabilized material were also included in the study. MONEL^{®} alloy 400 coupons were added to the 300 ppm PTAP stabilized sample.

**Table 4. 90 Day Storage Stability Study, Low levels of MEHQ and PTAP as Stabilizers in 1,1,3,3-Tetrachloropropene**

| Stabilizer | % HCl | | | |
|---|---|---|---|---|
| | Day 0 | Day 30 | Day 60 | Day 90 |
| 10.2 ppm MEHQ | 0.0174 | 0.0065 | 0.0012 | 0.0069 |
| 19.8 ppm MEHQ | 0.0134 | 0.0027 | N/A | 0.005 |
| 51 ppm MEHQ | 0.0131 | 0.0057 | 0.0024 | 0.0069 |
| 19.9 ppm PTAP | 0.0134 | 0.0057 | 0.0025 | 0.0069 |
| 303 ppm PTAP MONEL^{®} alloy | 0.0047 | 0.0052 | 0.0037 | 0.0074 |
| Unstabilised | 0.0136 | 0.0323 | 0.0208 | 0.0233 |

No increases in HCl values were seen over the 90-day storage period, even for the 10 ppm MEHQ. In fact, the HCl values are lower than the starting materials except in the case of the unstabilized material. No coupon corrosion or solution color was observed for the 300 PTAP with MONEL^{®} sample.

### Example 4. Effect of MEHQ and PTAP Stabilizing 1,1,2,3-Tetrachloropropene Stored Under Various Conditions

1,1,2,3-tetrachloropropene compositions were subjected to a variety of storage conditions and evaluated for evidence of breakdown or reactivity of the composition. MEHQ and PTAP were used to stabilize the 1,1,2,3-tetrachloropropene containing compositions.

Samples of 1,1,2,3 tetrachloropropene were stored in the absence of light at ambient temperature, approximately 25 °C, over a 20-day period. Stabilizer levels included unstabilized, and various combinations of MEHQ and PTAP at the concentrations as shown in Table 5. No metals were in contact with the samples and no efforts were made to exclude air prior to scaling the sample bottles for storage.

**Table 5 20 Day Storage Stability Study, Low levels of Combination of MEHQ and PTAP as Stabilizers in 1,1,2,3-Tetrachloropropene**

| Stabilizers | HCI, ppm | |
|---|---|---|
| | Day 0 | Day 20 |
| Unstabilized | 68 | 2057 |
| 1 ppm PTAP and 2 ppm MEHQ | 68 | 122 |
| 5 ppm PTAP and 5 ppm MEHQ | 68 | 106 |
| 12 ppm PTAP and 13 ppm MEHQ | 68 | 111 |
| 18 ppm PTAP and 19 ppm MEHQ | 68 | 107 |
| 28 ppm PTAP and 30 ppm MEHQ | 68 | 96 |

The unstabilized sample showed a 30 times increase in acidity levels from the starting materials whereas the stabilized samples showed less than twice the increase over the storage time.

Other examples of the efficacy of MEHQ as a stabilizer for chlorinated propenes include very low increases in acidity when samples are stored in a refrigerator at 5 °C and in the absence of light. This is shown in Table 6. No metals were present in the samples.

**Table 6 Study of MEHQ as Stabilizer for 1,1,2,3-Tetrachloropropene**

| MEHQ Concentration | Storage Time, Days | Increase in Acidity as HCl, ppm |
|---|---|---|
| 12 ppm | 101 | 8 |
| 26 ppm | 54 | 25 |

Unstabilized samples stored under similar conditions arc known to generate HCl much more rapidly that seen in the above stabilized samples.

## Claims

1. A composition comprising a chlorinated propene and at least one compound selected from the group consisting of *p*-methoxyphenol and p-tert-amylphenol, wherein the chlorinated propene is a compound according to the following formula:
C(H)₃₋ₓ(Cl)ₓ-C(H)_{1-y}(C)_{y}=C(H)_{2-z}(Cl)_{z}
wherein:
x is 0, 1, 2, or 3;
y is 0 or 1; and
z is 0, 1, or 2;
provided that at least one of x, y, and z is other than zero.

2. A composition according to claim 1, further comprising an additional phenol compound selected from the group consisting of isopropyl-meta cresol, 4,4'-methylenebis(2,6-di-*tert*-butylphenol); 4,4'-bis(2,6-di-*tert*-butylphenol); 2,2-biphenyldiols, 4,4-biphenyldiols; derivatives of 2,2- and 4,4-biphenyldiols; 2,2'-methylenebis(4-ethyl-6-*tert*-butylphenol); 2,2'-methylenebis(4-methyl-6-tertbutylphenol); 4,4,-butylidenebis(3-methyl-6-*tert*-butylphenol); 4,4,-isopropylidenebis(2,6-di-*tert*-butylphenol); 2,2'-methylenebis(4-methyl-6-nonylphenol); 2,2'-isobutylidenebis(4,6-dimethylphenol); 2,2'-methylenebis(4-methyl-6-cyclohexylphenol); 2,6-di-*tert*-butyl-4-methyl-phenol; 2,6-di-*tert*-butyl-4-ethylphenol; 2,4-dimethyl-6-*tert*-butylphenol; 2,6-di-*tert*-α-dimethylamino-*p-*cresol; 2,6-di-*tert*-butyl-4-(N,N'-dimethylaminomethyl)phenol; 4,4'-thiobis(2-methyl-6-*tert*-butylphenol); 4,4'-thiobis (3-methyl-6-*tert*-butylphenol); 2,2'-thiobis (4-methyl-6-*tert*-butylphenol); tocopherol; hydroquinone; *tert*-butyl hydroquinone.

3. A composition according to claim 1 or 2, comprising *p*-methoxyphenol.

4. A composition according to claim 1 or 2, comprising *p*-*tert*-amylphenol.

5. A composition according to any one of claims 1 to 4, wherein the chlorinated propene is a trichloropropene or a tetrachloropropene.

6. A composition according to claim 5, wherein the trichloropropene or tetrachloropropene is selected from the group consisting of 1,1,3-trichloropropene, 1,1,2,3-tetrachloropropene, 2,3,3,3-tetrachloropropene, 1,1,3,3-tetrachloropropene, 1,3,3,3-tetrachloropropene and (*cis* or *trans*)-1,2,3,3-tetrachloropropene.

7. A composition according to claim 5, wherein the chlorinated propene is a tetrachloropropene, wherein the tetrachloropropene is 1,1,2,3-tetrachloropropene.

8. A composition according to any one of claims 1 to 7 consisting essentially of the chlorinated propene and the at least one compound selected from the group consisting of *p*-methoxyphenol and *p-tert*-amylphenol.

9. A composition according to any one of claims 1 to 8 further comprising HF.

10. A composition according to claim 9 further comprising Cl₂ and SbCl₅.

11. A composition according to any one of claims 1 to 10 for use in the synthesis of 2,3,3,3-tetrafluoropropene, wherein the chlorinated propene is 1,1,2,3-tetrachloropropene.

12. An article comprising a container and a composition according to any one of claims 1 to 11 contained within said container.

13. An article according to claim 12, wherein the container is a metal container, wherein the metal optionally comprises stainless steel or a MONEL metal, and wherein the container is optionally lined with a polymeric coating such as a coating comprising a phenolic or epoxy resin.

14. A method of making a fluorinated propene, the method comprising:
(1) providing a composition according to any one of claims 1 to 11; and
(2) subjecting the composition to reaction conditions sufficient to effect a reaction wherein at least one chlorine atom of the chlorinated propene is substituted by fluorine to provide a fluorinated propene.

15. A method according to claim 14 comprising reacting the chlorinated propene with HF, wherein the chlorinated propene is 1,1,2,3-tetrachloropropene and the fluorinated propene formed is 2,3,3,3-tetrafluoropropene.

## Patentansprüche

1. Zusammensetzung, die ein chloriertes Propen und mindestens eine Verbindung, ausgewählt aus der Gruppe, bestehend aus *p*-Methoxyphenol und *p*-tert-Amylphenol, umfasst, wobei das chlorierte Propen eine Verbindung gemäß der folgenden Formel ist:
C(H)₃₋ₓ(Cl)ₓ-C(H)_{1-y}(Cl)_{y}=C(H)_{2-z}(Cl)_{z}
worin:
x 0, 1, 2 oder 3 ist;
y 0 oder 1 ist; und
z 0, 1 oder 2 ist;
mit der Maßgabe, dass mindestens eines von x, y und z anders als Null ist.

2. Zusammensetzung gemäß Anspruch 1, die weiterhin eine zusätzliche Phenolverbindung, ausgewählt aus der Gruppe, bestehend aus Isopropyl-meta-kresol, 4,4'-Methylenbis(2,6-di-*tert-*butylphenol); 4,4'-Bis(2,6-di-tert-butylphenol); 2,2-Biphenyldiolen, 4,4-Biphenyldiolen; Derivativen von 2,2- und 4,4-Biphenyldiolen; 2,2'-Methylenbis(4-ethyl-6-*tert*-butylphenol); 2,2'-Methylenbis(4-methyl-6-*tert*-butylphenol); 4,4,-Butylidenbis(3-methyl-6-*tert*-butylphenol); 4,4,-Isopropylidenbis(2,6-di-*tert*-butylphenol); 2,2'-Methylenbis(4-methyl-6-nonylphenol); 2,2'-Isobutylidenbis(4,6-dimethylphenol); 2,2'-Methylenbis(4-methyl-6-cyclohexylphenol); 2,6-Di-tert-butyl-4-methylphenol; 2,6-Di-*tert*-butyl-4-ethylphenol; 2,4-Dimethyl-6-*tert*-butylphenol; 2,6-Di-*tert*-α-dimethylamino-*p-*kresol; 2,6-Di-*tert*-butyl-4-(N,N'-dimethylaminomethyl)phenol; 4,4'-Thiobis(2-methyl-6-tert-butylphenol); 4,4'-Thiobis(3-methyl-6-*tert*-butylphenol); 2,2'-Thiobis(4-methyl-6-*tert-*butylphenol); Tocopherol; Hydrochinon; *tert*-Butylhydrochinon, umfasst.

3. Zusammensetzung gemäß Anspruch 1 oder 2, die *p*-Methoxyphenol umfasst.

4. Zusammensetzung gemäß Anspruch 1 oder 2, die p-tert-Amylphenol umfasst.

5. Zusammensetzung gemäß einem der Ansprüche 1 bis 4, wobei das chlorierte Propen ein Trichlorpropen oder ein Tetrachlorpropen ist.

6. Zusammensetzung gemäß Anspruch 5, wobei das Trichlorpropen oder Tetrachlorpropen ausgewählt ist aus der Gruppe, bestehend aus 1,1,3-Trichlorpropen, 1,1,2,3-Tetrachlorpropen, 2,3,3,3-Tetrachlorpropen, 1,1,3,3-Tetrachlorpropen, 1,3,3,3-Tetrachlorpropen und (*cis* oder *trans*)-1,2,3,3-Tetrachlorpropen.

7. Zusammensetzung gemäß Anspruch 5, wobei das chlorierte Propen ein Tetrachlorpropen ist, wobei das Tetrachlorpropen 1,1,2,3-Tetrachlorpropen ist.

8. Zusammensetzung gemäß einem der Ansprüche 1 bis 7, die im Wesentlichen aus dem chlorierten Propen und der mindestens einen Verbindung, ausgewählt aus der Gruppe, bestehend aus *p*-Methoxyphenol und *p*-tert-Amylphenol, besteht.

9. Zusammensetzung gemäß einem der Ansprüche 1 bis 8, die weiterhin HF umfasst.

10. Zusammensetzung gemäß Anspruch 9, die weiterhin Cl₂ und SbCl₅ umfasst.

11. Zusammensetzung gemäß einem der Ansprüche 1 bis 10 zur Verwendung in der Synthese von 2,3,3,3-Tetrafluorpropen, wobei das chlorierte Propen 1,1,2,3-Tetrachlorpropen ist.

12. Artikel, der einen Behälter und eine Zusammensetzung gemäß einem der Ansprüche 1 bis 11, die innerhalb des genannten Behälters enthalten ist, umfasst.

13. Artikel gemäß Anspruch 12, wobei der Behälter ein Metallbehälter ist, wobei das Metall optional Edelstahl oder ein MONEL-Metall umfasst, und wobei der Behälter optional mit einem polymeren Überzug, wie einem Überzug, der ein Phenol- oder Epoxyharz umfasst, ausgekleidet ist.

14. Verfahren zur Herstellung eines fluorierten Propens, wobei das Verfahren umfasst:
(1) Bereitstellen einer Zusammensetzung gemäß einem der Ansprüche 1 bis 11; und
(2) Unterwerfen der Zusammensetzung Reaktionsbedingungen, die ausreichend sind, um eine Reaktion zu bewirken, wobei mindestens ein Chloratom des chlorierten Propens unter Erhalt eines fluorierten Propens durch Fluor substituiert wird.

15. Verfahren gemäß Anspruch 14, das Umsetzen des chlorierten Propens mit HF umfasst, wobei das chlorierte Propen 1,1,2,3-Tetrachlorpropen ist und das gebildete fluorierte Propen 2,3,3,3-Tetrafluorpropen ist.

## Revendications

1. Composition comprenant un propène chloré et au moins un composé choisi dans le groupe constitué par le p-méthoxyphénol et le p-tert-amylphénol, où le propène chloré est un composé selon la formule suivante :
C(H)₃₋ₓ(Cl)ₓ-C(H)_{1-y}(Cl)_{y}=C(H)_{2-z}(Cl)_{z}
dans laquelle :
x vaut 0, 1, 2 ou 3 ;
y vaut 0 ou 1 ; et
z vaut 0, 1 ou 2 ;
à condition qu'au moins l'un de x, y et z soit différent de zéro.

2. Composition selon la revendication 1, comprenant en outre un composé phénol supplémentaire choisi dans le groupe constitué par l'isopropyl-métacrésol, le 4,4'-méthylènebis(2,6-di-tert-butylphénol) ; le 4,4'-bis(2,6-di-tert-butylphénol) ; les 2,2-biphényldiols, les 4,4-biphényldiols ; des dérivés des 2,2- et 4,4-biphényldiols ; le 2,2'-méthylènebis(4-éthyl-6-tert-butylphénol) ; le 2,2'-méthylènebis(4-méthyl-6-tert-butylphénol) ; le 4,4'-butylidènebis(3-méthyl-6-tert-butylphénol) ; le 4,4'-isopropylidène-bis(2,6-di-tert-butylphénol) ; le 2,2'-méthylènebis(4-méthyl-6-nonylphénol) ; le 2,2'-isobutylidènebis(4,6-diméthylphénol) ; le 2,2'-méthylènebis(4-méthyl-6-cyclohexylphénol) ; le 2,6-di-tert-butyl-4-méthyl-phénol ; le 2,6-di-tert-butyl-4-éthylphénol ; le 2,4-diméthyl-6-tert-butylphénol ; le 2,6-di-tert-α-diméthylamino-p-crésol ; le 2,6-di-tert-butyl-4-(N,N'-diméthylaminométhyl)phénol ; le 4,4'-thiobis(2-méthyl-6-tert-butylphénol) ; le 4,4'-thiobis(3-méthyl-6-tert-butylphénol) ; le 2,2'-thiobis(4-méthyl-6-tert-butyl-phénol) ; le tocophérol ; l'hydroquinone ; la tert-butyl-hydroquinone.

3. Composition selon la revendication 1 ou 2, comprenant du p-méthoxyphénol.

4. Composition selon la revendication 1 ou 2, comprenant du p-tert-amylphénol.

5. Composition selon l'une quelconque des revendications 1 à 4, dans laquelle le propène chloré est un trichloropropène ou un tétrachloropropène.

6. Composition selon la revendication 5, dans laquelle le trichloropropène ou le tétrachloropropène est choisi dans le groupe constitué par le 1,1,3-trichloropropène, le 1,1,2,3-tétrachloropropène, le 2,3,3,3-tétrachloropropène, le 1,1,3,3-tétrachloropropène, le 1,3,3,3-tétrachloropropène et le (cis ou trans)-1,2,3,3-tétrachloropropène.

7. Composition selon la revendication 5, dans laquelle le propène chloré est un tétrachloropropène, où le tétrachloropropène est le 1,1,2,3-tétrachloropropène.

8. Composition selon l'une quelconque des revendications 1 à 7, constituée essentiellement du propène chloré et dudit au moins un composé choisi dans le groupe constitué par le p-méthoxyphénol et le p-tert-amylphénol.

9. Composition selon l'une quelconque des revendications 1 à 8, comprenant en outre du HF.

10. Composition selon la revendication 9, comprenant en outre du Cl₂ et du SbCl₅.

11. Composition selon l'une quelconque des revendications 1 à 10, pour une utilisation dans la synthèse de 2,3,3,3-tétrafluoropropène, où le propène chloré est le 1,1,2,3-tétrachloropropène.

12. Article comprenant un récipient et une composition selon l'une quelconque des revendications 1 à 11 contenue dans ledit récipient.

13. Article selon la revendication 12, dans lequel le récipient est un récipient en métal, où le métal comprend éventuellement de l'acier inoxydable ou un métal monel, et où le récipient est éventuellement revêtu d'un enrobage polymère comme un enrobage comprenant une résine phénolique ou époxy.

14. Procédé de fabrication d'un propène fluoré, le procédé comprenant :
(1) la fourniture d'une composition selon l'une quelconque des revendications 1 à 11 ; et
(2) la soumission de la composition à des conditions réactionnelles suffisantes pour effectuer une réaction dans laquelle au moins un atome de chlore du propène chloré est substitué par du fluor pour fournir un propène fluoré.

15. Procédé selon la revendication 14, comprenant la réaction du propène chloré avec du HF, où le propène chloré est le 1,1,2,3-tétrachloropropène et le propène fluoré est le 2,3,3,3-tétrafluoropropène.
